# EUROPEAN PATENT APPLICATION

(11) **EP 4 520 265 A1**
(43) Date of publication of application: **12.03.2025**
(21) Application number: 23854669.1
(22) Date of filing: 20.02.2023
(51) Int. Cl.: A61B 5/11, A61B 5/0245, A61B 5/08

(54) **BIOLOGICAL STATE MEASUREMENT DEVICE, BIOLOGICAL STATE MEASUREMENT METHOD, PROGRAM, AND BIOLOGICAL STATE MEASUREMENT SYSTEM**

(30) Priority: 17.08.2022 JP 2022130000
(71) Applicant: FINGGAL LINK CO., LTD., Taito-ku Tokyo 111-0041 (JP)
(72) Inventor: TAMURA, Yasuhiro, Tokyo 111-0041 (JP)
(74) Representative: Plougmann Vingtoft a/s
(86) International application number: PCT/JP2023/006015
(87) International publication number: WO 2024/038629

(57) **Abstract**

This biological state measurement device 1 has: a signal acquisition unit 153 for acquiring a reflection signal produced by reflection, by a subject U, of a chirp signal in the frequency band of the millimeter wave band or higher transmitted every predetermined frame time, and a signal conversion unit 154 for converting the reflection signal every frame time into a complex signal in the frequency domain; a frequency selection unit 155 for selecting a frequency corresponding to a frequency component that has a relatively high intensity from among a plurality of frequency components included in the complex signal; and a measurement unit 156 for identifying the biological state of the subject U, on the basis of a change in the phase of the frequency component corresponding to the frequency selected by the frequency selection unit 155, over a frequency fixation period longer than the frame time.

## Description

### TECHNICAL FIELD

The present invention relates to a biological condition measurement apparatus, a biological condition measurement method, a program, and a biological condition measurement system for measuring a biological condition of humans.

### BACKGROUND OF THE INVENTION

There are conventionally known methods of measuring heart rates by transmitting millimeter wave signals, and analyzing reflected wave signals generated by the reflection of the transmitted millimeter wave signals on a human body (e.g. see Patent Document 1).

### PRIOR ART

### PATENT DOCUMENT

[Patent Document 1] Japanese Patent Application Publication No. 2016-214876

### SUMMARY OF INVENTION

### PROBLEMS TO BE SOLVED BY THE INVENTION

The transmitted millimeter wave signals are reflected by various objects in the space, generating different reflection signals. When a large number of these reflection signals are analyzed, the problem had arisen that the computational load required to measure biological conditions such as heart rate and respiration becomes enormous.

The present invention has been made in view of these matters, and aims to reduce the computational load for measuring biological conditions using reflection signals.

### MEANS FOR SOLVING THE PROBLEMS

A biological condition measurement apparatus according to a first aspect of the present invention includes: a signal acquiring section that acquires a reflection signal generated by reflection, on a measurement subject, of a chirp signal in a frequency band equal to or higher than a millimeter wave band, transmitted at each predetermined frame time; a signal converting section that converts the reflection signal into a complex signal in a frequency domain for each frame time; a frequency selection section that selects a frequency corresponding to a frequency component with relatively high intensity from among a plurality of frequency components included in the complex signal; and a measuring section that identifies biological conditions of the measurement subject on the basis of changes in a phase of the frequency component corresponding to the frequency selected by the frequency selection section over a frequency fixing period which is longer than the frame time.

The measuring section may generate a phase signal by arranging phases of the frequency component in time series over the frequency fixing period, and identify the biological conditions on the basis of a frequency component included in a cycle range or a frequency range corresponding to the biological conditions in the phase signal.

The measuring section may identify a time interval between peaks in the phase signal as a cycle of the biological conditions when the time interval falls within a range assumed as the cycle of the biological condition to be identified.

The measuring section may convert the phase signal into a frequency-domain signal, identifies, as respiratory frequency, a frequency of a frequency component with a first intensity or higher among a plurality of frequency components included in a respiratory frequency band, and identify, as a heartbeat frequency, a frequency of a frequency component with a second intensity or higher among a plurality of frequency components included in a heartbeat frequency band.

The measuring section may identify a heart rate on the basis of the phase signal over the frequency fixing period, and identify a respiratory rate on the basis of the phase signal that spans 1.5 times or more and 2.5 times or less the frequency fixing period. The frequency fixing period may be 5 seconds or more and less than 10 seconds.

The measuring section may correct the phase signal so as to reduce discontinuity of the phase signal at a timing at which a frequency selected by the frequency selection section is switched.

The frequency selection section may select a frequency corresponding to a frequency component with relatively high intensity at each frequency fixing period. The frequency selection section may select a frequency corresponding to a frequency component with the highest intensity from among the plurality of frequency components, for example.

The signal acquiring section may acquire the reflection signal generated by reflection on a plurality of the subjects; and the frequency selection section may select a frequency corresponding to the number of frequency components corresponding to the number of measurement subjects from among the plurality of frequency components.

The biological condition measurement apparatus may include a storage section that stores a frequency corresponding to the frequency component selected by the frequency selection section, and the measuring section may identify the biological conditions of the measurement subject on the basis of changes in the phase of the frequency component corresponding to the frequency stored in the storage section.

A biological condition measurement method according to a second aspect of the present invention is executed by a computer and including the steps of: acquiring a reflection signal generated by reflection, on a measurement subject, of a chirp signal in a frequency band equal to or higher than a millimeter wave band, transmitted at each predetermined frame time; a signal converting section that converts the reflection signal into a complex signal in a frequency domain for each frame time; selecting a frequency corresponding to a frequency component with a relatively high intensity from among a plurality of frequency components included in the complex signal; and identifying biological conditions of the measurement subject on the basis of changes in a phase of the frequency component corresponding the selected frequency over a frequency fixing period which is longer than the frame time.

A program according to a third aspect of the present invention causes a computer to execute the steps of: acquiring a reflection signal generated by reflection, on a measurement subject, of a chirp signal in a frequency band equal to or higher than a millimeter wave band, transmitted at each predetermined frame time; a signal converting section that converts the reflection signal into a complex signal in a frequency domain for each frame time; selecting a frequency corresponding to a frequency component with a relatively high intensity from among a plurality of frequency components included in the complex signal; and identifying biological conditions of the measurement subject on the basis of changes in a phase of the frequency component corresponding to the selected frequency over a frequency fixing period which is longer than the frame time.

A biological condition measurement system according to a fourth aspect of the present invention includes: a biological condition measurement apparatus; and an external apparatus that can communicate with the biological condition measurement apparatus, wherein the biological condition measurement apparatus includes: a signal acquiring section that acquires a reflection signal generated by reflection, on a measurement subject, of a chirp signal in a frequency band equal to or higher than a millimeter wave band, transmitted at each predetermined frame time; the biological condition measurement apparatus or the external apparatus has: a signal converting section that converts the reflection signal into a complex signal in a frequency domain for each frame time; a frequency selection section that selects a frequency corresponding to a frequency component with relatively high intensity from among a plurality of frequency components included in the complex signal; and a measuring section that identifies biological conditions of the measurement subject on the basis of changes in a phase of the frequency component corresponding to the frequency selected by the frequency selection section over a frequency fixing period which is longer than the frame time.

### EFFECT OF THE INVENTION

The present invention attains an effect that makes it possible to reduce the computational load for measuring biological conditions using reflection signals.

### BRIEF DESCRIPTION OF THE DRAWINGS

FIG. 1 shows an outline of a biological condition measurement system S1.
FIG. 2 shows an example of a screen representing measurement results displayed on an information terminal 2.
FIG. 3 shows another example of a screen representing measurement results displayed on the information terminal 2.
FIG. 4 shows a configuration of a biological condition measurement apparatus 1.
FIG. 5 illustrates transmission areas of transmitting sections 11 and reception areas of receiving sections 12.
FIG. 6 shows an example of a frequency fixing period.
FIG. 7 illustrates an operation of a measuring section 156.
FIG. 8 illustrates the operation of the measuring section 156.
FIG. 9 illustrates a process in which the measuring section 156 tracks biological conditions of a measurement subject U.
FIG. 10 is a flowchart showing an outline of a processing procedure in the biological condition measurement apparatus 1.
FIG. 11 is a flowchart showing details of a process of analyzing reflection signals in the biological condition measurement apparatus 1.
FIG. 12 shows a configuration of a biological condition measurement system S2 according to a modification example.

### DETAILED DESCRIPTION OF THE INVENTION

### [Outline of biological condition measurement system S1]

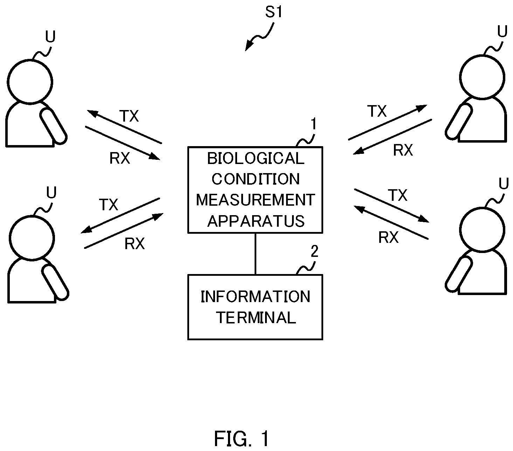

FIG. 1 shows an outline of a biological condition measurement system S1. The biological condition measurement system S1 is a system for measuring biological conditions of humans. The biological conditions are heart rates or respiratory rates, or motions of body parts of humans. The biological conditions are represented by change amounts per unit time of the heart rates, the respiratory rates, or the motions, for example.

The biological condition measurement system S1 includes a biological condition measurement apparatus 1 and an information terminal 2. The biological condition measurement apparatus 1 can measure biological conditions of a human (a measurement subject U in FIG. 1) within an area where radio waves generated by the biological condition measurement apparatus 1 can reach. The information terminal 2 is a terminal for displaying results of measurement by the biological condition measurement apparatus 1, controlling the biological condition measurement apparatus 1, and so on, and is a computer, a tablet, or a smartphone, for example.

The biological condition measurement apparatus 1 transmits, as a transmission signal, radio waves with frequencies equal to or higher than the millimeter wave band in a plurality of directions, and receives reflection signals generated by the reflection of the transmitted radio waves on nearby objects. The biological condition measurement apparatus 1 identifies the distances to the objects having reflected the transmission signal on the basis of propagation times from the transmission of the transmission signal until the reception of the reflection signal. A human body has tissues that easily reflect radio waves and tissues that do not easily reflect radio waves, and, by identifying the distance to a tissue that easily reflects radio waves, the biological condition measurement apparatus 1 can identify motions of the tissue. For example, the biological condition measurement apparatus 1 measures heart rates by identifying cardiac motions, and measures respiratory rates by identifying pleural motions.

The transmission signal is, for example, a 60 GHz band signal. Since the wavelength of the 60 GHz signal is 5 mm, a change of 5 mm in the position of an object reflecting the transmission signal corresponds to a phase change of 2π. The biological condition measurement apparatus 1 can detect minute changes as small as 5 mm or less in parts such as the heart or lungs by identifying a change in the phase of the reflection signal.

The biological condition measurement apparatus 1 transmits, as a transmission signal, a chirp signal with frequencies that change over time. In a case where the biological condition measurement apparatus 1 transmits a chirp signal, the biological condition measurement apparatus 1 transforms a reflection signal into a frequency-domain signal by the Fourier transform, and identifies a frequency included in the reflection signal. Thereby, the biological condition measurement apparatus 1 can measure the propagation time from the transmission of a signal with the frequency until reception of the signal highly precisely.

The chirp signal is a signal whose frequency changes over time. Therefore, a reflection signal generated after the reflection of the chirp signal by the object includes signals of numerous frequencies included in the chirp signal's frequency band. When this reflection signal is converted into a frequency-domain signal and then analyzed, the computational load required for analysis becomes enormous if all signals in a wide frequency range are analyzed.

The inventor of the present application has found that the intensity (that is, amplitude or power) of each frequency component included in the reflection signal varies according to the frequency, and that the frequency with higher intensity varies depending on the position of the measurement subject U. Based on this principle, the biological condition measurement apparatus 1 selects a frequency corresponding to a frequency component with a relatively high intensity from among a plurality of frequency components included in the reflection signal, and identifies biological conditions of the measurement subject U on the basis of changes in the phase of the frequency component corresponding to the selected frequency over a predetermined frequency fixing period. Although details of the frequency fixing period will be described later, it refers to a length of time set in advance to be longer than a frame time of an FFT (Fast Fourier Transform) process executed by the biological condition measurement apparatus 1, and is, for example, 5 seconds or more and less than 10 seconds.

By being configured in this way, the biological condition measurement apparatus 1 can significantly reduce the computational load while improving accuracy of the analysis. Further, since the frequency to be analyzed is periodically reselected, the accuracy of the analysis can be maintained at a high level even when the position of the measurement subject U changes.

### [Display screen of measurement results]

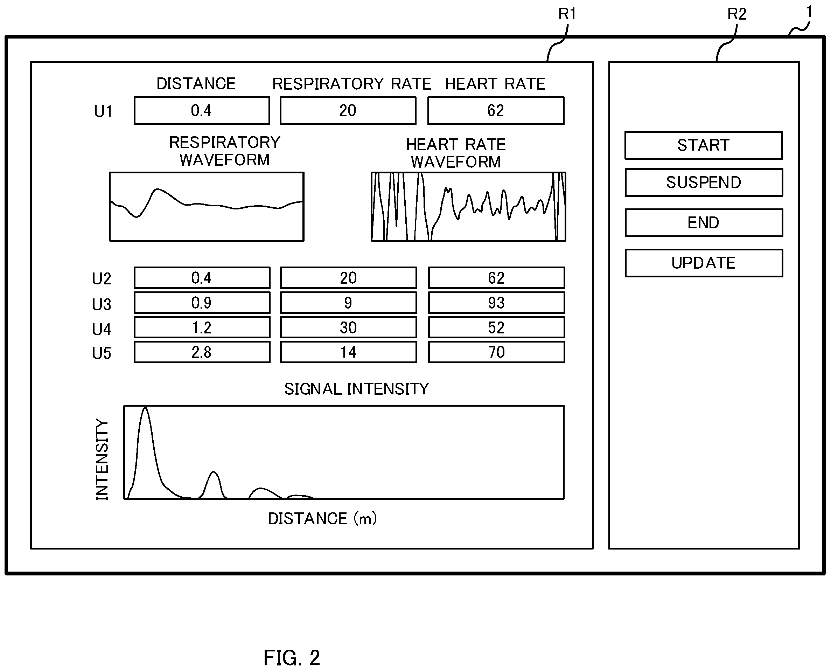

FIG. 2 shows an example of a screen representing measurement results displayed on the information terminal 2. The screen shown in FIG. 2 includes a measurement result display region R1 and an operation region R2.

In the uppermost portion of the region R1, the distance from the biological condition measurement apparatus 1 to a measurement subject U1, who is the closest measurement subject U from the biological condition measurement apparatus 1, and the respiratory rate and heart rate of the measurement subject U1 are depicted. In addition, the respiratory waveform and the heart rate waveform of the measurement subject U1 are also depicted. Below the respiratory waveform and the heart rate waveform, the distances to a plurality of other measurement subjects U, and the respiratory rates and the heart rates of the measurement subjects U are depicted in association with the respective measurement subjects U. Further, in the region R1, a figure depicting the relationship between the distances to the measurement subjects U and the intensities of received reflection signal also is depicted.

In the region R2, images of operation buttons for accepting operations for starting, suspending, and ending measurement are displayed. An "update" button in the region R2 is a button for updating measurement results being displayed in the region R1 to the latest measurement results.

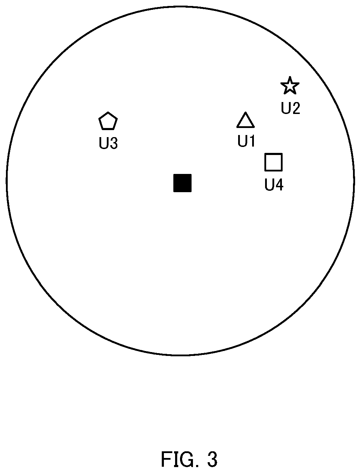

FIG. 3 shows another example of the screen representing measurement results displayed on the information terminal 2. FIG. 3 shows the positions of a plurality of the measurement subjects U whose biological conditions were measured. A black quadrilateral in FIG. 3 represents the position of the biological condition measurement apparatus 1, and other white figures represent the positions of the plurality of measurement subjects U having reflected the transmission signal. The biological condition measurement apparatus 1 may include the image depicted in FIG. 3 in the screen depicted in FIG. 2 or may cause the information terminal 2 to display the image depicted in FIG. 3 in a case where predetermined operation is performed on the screen depicted in FIG. 2.

### [Configuration of the biological condition measurement apparatus 1]

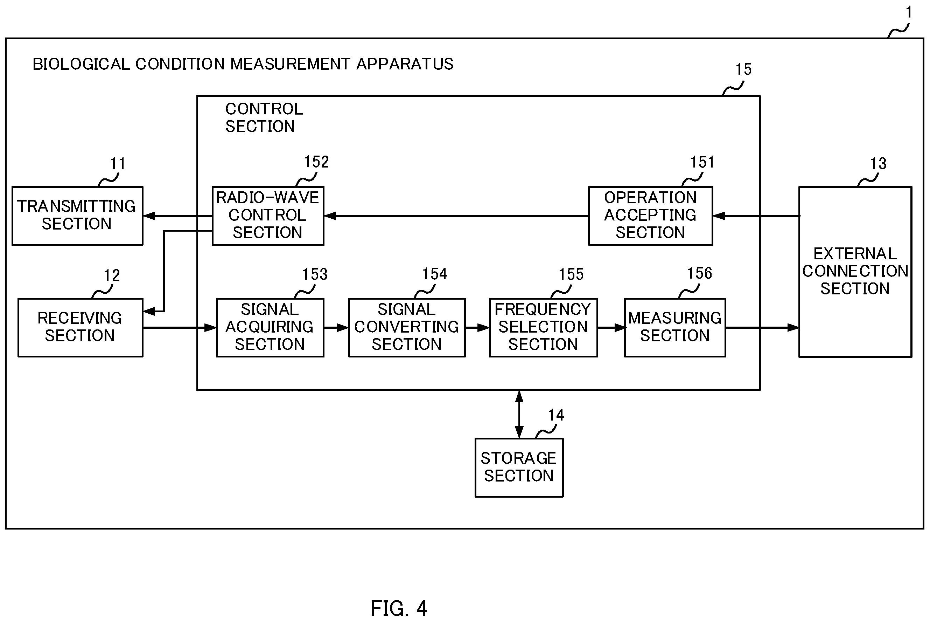

FIG. 4 shows a configuration of the biological condition measurement apparatus 1. The biological condition measurement apparatus 1 includes a transmitting section 11, a receiving section 12, an external connection section 13, a storage section 14, and a control section 15. The control section 15 includes an operation accepting section 151, a radio-wave control section 152, a signal acquiring section 153, a signal converting section 154, a frequency selection section 155, and a measuring section 156.

The transmitting section 11 transmits transmission signals in a frequency band equal to or higher than the millimeter wave band at predetermined time intervals under control of the radio-wave control section 152. For example, the transmitting section 11 transmits chirp signals in cycles of 12.5 milliseconds. The transmitting section 11 has a signal generating circuit that generates chirp signals, and an antenna for transmitting the transmission signals as radio waves. For example, the biological condition measurement apparatus 1 may have a plurality of the transmitting sections 11 that transmit transmission signals toward different areas at mutually different timings.

The transmitting section 11 transmits transmission signals at preset time intervals shorter than half of the minimum value of a cycle in which biological conditions change. For example, in a case where it is assumed that the maximum value of heart rates is 150 times/minute, the minimum value of the fluctuation cycle of the heart rates is 0.4 seconds. In view of this, the transmitting section 11 transmits transmission signals at time intervals shorter than 0.2 seconds. As one example, the transmitting section 11 according to the present embodiment transmits transmission signals in cycles of 12.5 milliseconds (corresponding to 80 Hz). The transmission of transmission signals by the transmitting section 11 at these time intervals allows the biological condition measurement apparatus 1 to identify changes in the condition of body parts of the measurement subject U on the basis of the reflection signals received by the receiving section 12 at said time intervals.

The length of transmission signals transmitted by the transmitting section 11 can be any length, but it is desirable if the length is sufficiently shorter than time intervals at which the transmission signals are transmitted, and is equal to or shorter than 2 milliseconds, for example. In a case where the biological condition measurement apparatus 1 has a plurality of transmitting sections 11, the length of transmission signals transmitted by the transmitting sections 11 is determined on the basis of the number of the transmitting sections 11. Specifically, the length of the transmission signals is shorter than a length of time which is the quotient of the length of time intervals at which the transmission signals are transmitted, by the number of the transmitting sections 11. By setting the length of the transmission signals in this manner, the plurality of transmitting sections 11 can transmit the transmission signals at different timings.

The receiving section 12 receives a plurality of reflection signals generated by the reflection of transmission signals on a plurality of measurement subjects U. For example, there are a plurality of the receiving sections 12 that receive reflection signals coming from mutually different areas. The receiving sections 12 input the received reflection signals to the measuring section 156.

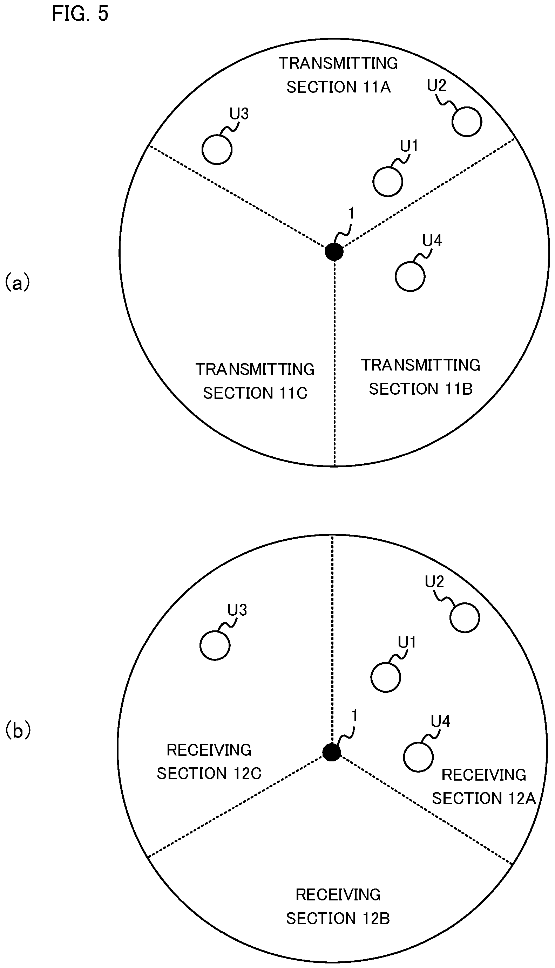

FIG. 5 illustrates transmission areas of the transmitting sections 11 and reception areas of the receiving sections 12. (a) in FIG. 5 schematically depicts an area toward which each of a transmitting section 11A, a transmitting section 11B and a transmitting section 11C transmits transmission signals, and the positions of a plurality of measurement subjects U, in a case where the biological condition measurement apparatus 1 has those transmitting sections 11. (b) in FIG. 5 schematically depicts an area from which each of a receiving section 12A, a receiving section 12B and a receiving section 12C receives reflection signals, and the positions of the plurality of measurement subjects U, in a case where the biological condition measurement apparatus 1 has those receiving sections 12.

In the example depicted in FIG. 5, the transmission area of each transmitting section 11 has a fan shape with ±60 degrees (120 degrees), and the reception area of each receiving section 12 has a fan shape with ±60 degrees (120 degrees). Although not depicted in FIG. 5, the transmitting sections 11 transmit transmission signals in a predetermined vertical range, and the receiving sections 12 receive reflection signals coming from a predetermined vertical range. For example, the predetermined ranges are ranges of ±15 degrees (30 degrees) centered on the horizontal direction.

As depicted in FIG. 5, it is desirable if the transmission areas of the transmitting sections 11 and the reception areas of the receiving section 12 do not match completely. The biological condition measurement apparatus 1 can identify that the measurement subject U is in an area where the transmission area of a transmitting section 11 that transmitted a transmission signal and the reception area of a receiving section 12 that received reflection signals based on the transmission signal overlap. Therefore, the number of areas where the transmission areas and the reception areas overlap increases in a case where the transmission areas and the reception areas do not match completely, thereby improving the resolution of the biological condition measurement apparatus 1 for identifying the position of the measurement subject U.

The external connection section 13 has a communication interface for transmitting and receiving data to and from the information terminal 2. For example, the external connection section 13 has a USB interface. The external connection section 13 receives operation data input on the information terminal 2, and inputs the received data to the operation accepting section 151. In addition, the external connection section 13 transmits, to the information terminal 2, data representing measurement results output by the output section 155.

The storage section 14 includes a storage medium such as a ROM (Read Only Memory) or a ROM (Random Access Memory. The storage section 14 stores programs to be executed by the control section 15. In addition, the storage section 14 stores reflection signals received by the receiving section 12, and measurement results generated by analysis of the reflection signals by the control section 15.

Furthermore, the storage section 14 stores a frequency selected by the frequency selection section 155 as the frequency corresponding to the frequency component used when the measuring section 156 analyzes the reflection signals. The frequency stored in the storage section 14 is updated at each predetermined cycle (for example, the frequency fixing period).

The control section 15 includes a CPU (Central Processing Unit), for example. The control section 15 functions as the operation accepting section 151, the radio-wave control section 152, the signal acquiring section 153, the signal converting section 154, the frequency selection section 155, and the measuring section 156 by executing programs stored in the storage section 14.

The operation accepting section 151 accepts operation by a user by receiving operation data input from the information terminal 2 via the external connection section 13. For example, the operation accepting section 151 accepts operation to start measurement, operation to suspend measurement, operation to end measurement, or operation to update measurement results that is input in the region R2 depicted in FIG. 2.

In addition, the operation accepting section 151 may accept operation to set a target area of identification of biological conditions by the measuring section 156 (hereinafter referred to as an "identification target area"). The operation accepting section 151 accepts setting of a range of distances to a target measurement subject U, who is the target of the identification of biological conditions, for example. The operation accepting section 151 may accept the setting of the target area of identification of biological conditions within a range of 0 degrees to 360 degrees, relative to a predetermined side (e.g., the front direction) of the biological condition measurement apparatus 1.

As one example, the operation accepting section 151 accepts setting of numerical values representing angles like "from 0 degrees to 90 degrees" or "from 270 degrees to 90 degrees." The operation accepting section 151 may accept setting of the target area of identification of biological conditions by causing the information terminal 2 to display a circular image like the one depicted in FIG. 3 via the external connection section 13, and accepting operation to circle the target area of the identification of biological conditions.

The radio-wave control section 152 controls the transmitting section 11 and the receiving section 12. The radio-wave control section 152 controls, for example, the timing at which the transmitting section 11 is caused to transmit the transmission signal. In addition, the radio-wave control section 152 controls a range in which the transmitting section 11 transmits the transmission signal and a range in which the receiving section 12 receives the reflection signal.

As one example, the radio-wave control section 152 controls the plurality of transmitting sections 11 such that one or more transmitting sections 11 corresponding to identification target areas accepted by the operation accepting section 151 in the plurality of transmitting sections 11 are caused to transmit transmission signals. In addition, the radio-wave control section 152 controls the plurality of receiving sections 12 such that one or more receiving sections 12 corresponding to identification target areas accepted by the operation accepting section 151 in the plurality of receiving sections 12 are caused to receive reflection signals. By the radio-wave control section 152 causing transmitting sections 11 corresponding to identification target areas to transmit transmission signals and causing receiving sections 12 corresponding to identification target areas to receive reflection signals in this manner, it becomes less likely to be influenced by unnecessary reflection signals coming from areas where there are no measurement subjects U, and accordingly the measurement precision improves.

After the measuring section 156 has identified the position of the measurement subject U, the radio-wave control section 152 may cause the transmitting sections 11 corresponding to the range corresponding to the position of the measurement subject U to transmit the transmission signals, and cause the transmitting sections 11 corresponding to all ranges to periodically transmit the transmission signals. By having the radio-wave control section 152 operate in this manner, reflection signals generated by the reflection of transmission signals on an object other than the measurement subject U are reduced, thereby reducing the load when the control section 15 analyzes the reflection signals.

By performing digital signal processing on reflection signals received by receiving section 12, the measuring section 156 measures a plurality of propagation times from transmission of transmission signals by transmitting sections 11 until reception of a plurality of reflection signals by the receiving sections 12 in order to identify the distance from the biological condition measurement apparatus 1 to the measurement subject U and the direction of the measurement subject U relative to the biological condition measurement apparatus 1. In a case where the biological condition measurement apparatus 1 includes a plurality of receiving sections 12, the measuring section 156 measures, for each receiving section 12, the propagation time from transmission of a transmission signal by a transmitting section 11 until reception of reflection signals by the plurality of receiving sections 12.

The measuring section 156 may measure, as the amount of change in the propagation time, the amount of change in the frequency of a chirp signal included in a transmission signal or the amount of change in the phase of a transmission signal. Since the amount of change in the frequency of the chirp signal or the amount of change in the phase of the transmission signal is equivalent to the amount of change in the propagation time, measurement of the amount of change in the frequency of the chirp signal or the amount of change in the phase of the transmission signal by the measuring section 156 can also be regarded as measurement of the amount of change in the propagation time.

In a case where the biological condition measurement apparatus 1 includes a plurality of transmitting sections 11, the measuring section 156 measures, for each transmitting section 11, the propagation time from transmission of a transmission signal by each of the plurality of transmitting sections 11 until reception of reflection signals by receiving sections 12. The measuring section 156 stores, in the storage section 14, propagation time data representing the measured propagation times in association with the respective transmitting sections 11. In a case where the biological condition measurement apparatus 1 includes a plurality of receiving sections 12, the measuring section 156 measures, for each receiving section 12, the propagation time from transmission of a transmission signal by a transmitting section 11 until reception of reflection signals by the plurality of receiving sections 12. The measuring section 156 stores, in the storage section 14, propagation time data representing the measured propagation times in association with the respective receiving sections 12.

In a case where the biological condition measurement apparatus 1 includes a plurality of transmitting sections 11 and a plurality of receiving sections 12, the measuring section 156 measures, for each combination of a transmitting section 11 and a receiving section 12, the propagation time from transmission of a transmission signal from each of the plurality of transmitting sections 11 until reception of the reflection signals by the plurality of the receiving sections 12. The measuring section 156 stores, in the storage section 14, propagation time data representing the measured propagation times in association with the combinations of the transmitting sections 11 and the receiving sections 12.

The signal acquiring section 153 acquires a reflection signal generated by the reflection, on the measurement subject U, of a transmission signal (i.e., chirp signal) in the frequency band equal to or higher than the millimeter wave band, transmitted at each predetermined frame time. The frame time corresponds to a length of time in which the signal converting section 154 executes the FFT to convert the reflection signal into a signal in the frequency domain. The signal converting section 154 may acquire a plurality of reflection signals generated by the reflection on a plurality of subjects. The signal converting section 154 inputs the acquired reflection signal to the signal converting section 154.

The signal converting section 154 converts the reflection signal into a complex signal in a frequency domain for each frame time. As one example, the transmission signal is quadrature modulation data, and the signal converting section 154 samples the I data and Q data in the received quadrature modulation data at each predetermined sampling time, and performs FFT operations on the sampled values to convert the reflection signal into the complex signal. The vector of the complex signal corresponds to the intensity and phase of the reflection signal.

The signal converting section 154 generates 512-byte digital data for the I data and 512-byte digital data for the Q data by sampling 256 times for each frame time. When the frame time is 12.5 milliseconds, the signal converting section 154 converts 80 frames into complex signals in one second, generating 5 × 2 × 80 bytes = 80 KB of data.

The frequency selection section 155 selects a frequency corresponding to a frequency component with relatively high intensity (that is, amplitude or power) from among a plurality of frequency components included in the complex signal. The frequency selection section 155 selects a frequency corresponding to the frequency component with the highest intensity from among the plurality of frequency components, for example. Specifically, the frequency selection section 155 selects a frequency corresponding to the complex signal with the highest intensity from among a plurality of complex signals, each corresponding to a plurality of frequencies generated by the signal converting section 154 in a given frame, and stores the selected frequency in the storage section 14.

The frequency stored in the storage section 14 in this manner is retained in the storage section 14 for the duration of the frequency fixing period. That is, the frequency selection section 155 selects a frequency corresponding to the frequency component with relatively high intensity at each frequency fixing period.

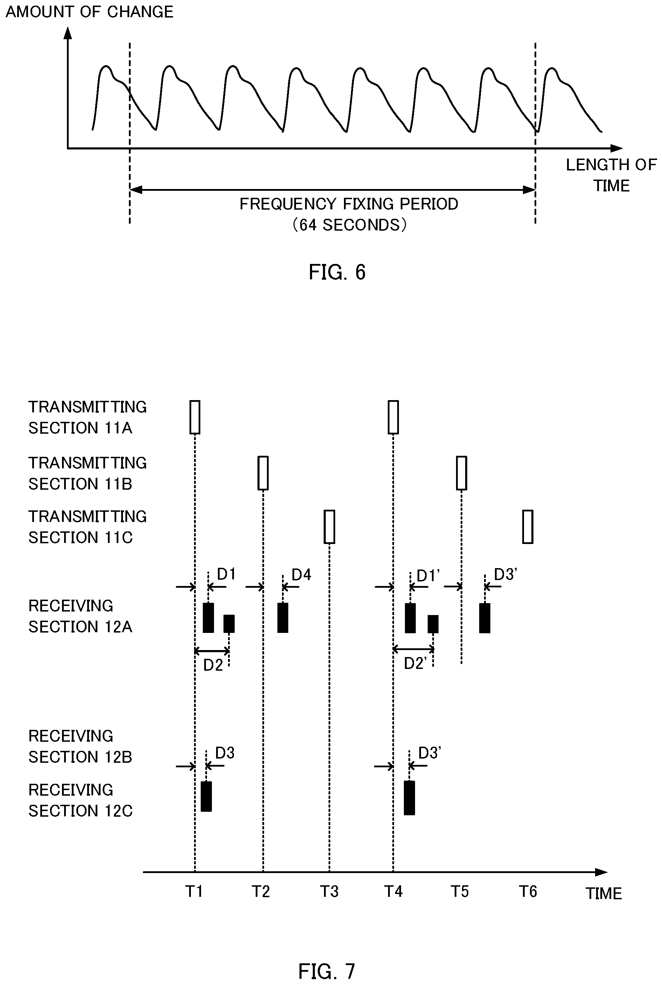

FIG. 6 shows an example of the frequency fixing period. In FIG. 6, the horizontal axis represents a length of time, and the vertical axis represents the amount of change in the biological conditions. For example, it is desirable for the frequency fixing period to be longer than the fluctuation cycle of the biological conditions to be measured, in order to ensure signal continuity. The frequency fixing period is 5 seconds or more, for example. In addition, when the position of the measurement subject U is changed, it is desirable to use a frequency suitable for the changed position, and thus the frequency fixing period is less than 10 seconds, for example. That is, the frequency fixing period is preferably 5 seconds or more and less than 10 seconds, and as an example as shown in FIG. 6, is 6.4 seconds, corresponding to 512 frames.

The frequency selection section 155 may select a plurality of frequencies corresponding to a predetermined number of frequencies, in order from the frequency with the highest intensity, from among the plurality of frequency components. The predetermined number is set via the operation accepting section 151 and stored in the storage section 14, and is, for example, four. By having the frequency selection section 155 select the plurality of frequencies, even if noise is superposed on some of the selected frequencies, resulting in reduction in the reliability, the measuring section 156 can use other frequencies to maintain accuracy in identifying the biological conditions.

The frequency selection section 155 may select, from among the plurality of frequency components, a frequency corresponding to the number of frequency components corresponding to the number of measurement subjects U. For example, the frequency selection section 155 selects the number of frequencies by multiplying the number of frequencies selected for one measurement subject U by the number of people set via the operation accepting section 151. Although details will be described later, by having the frequency selection section 155 operate in this manner, when the plurality of measurement subjects U are present at different positions, the measuring section 156 can identify biological conditions of each of the measurement subjects U with high accuracy by analyzing frequency components suitable for each of the measurement subjects U.

By analyzing the reflection signals, the measuring section 156 identifies three elements which are the distance between the biological condition measurement apparatus 1 and the measurement subject U, the direction of the measurement subject U relative to the biological condition measurement apparatus 1, and the movement speed of the measurement subject U, thereby identifying biological conditions of the measurement subject U. The measuring section 156 identifies the biological conditions of the measurement subject U on the basis of changes in the phase of the frequency component corresponding to the frequency selected by the frequency selection section 155 over the frequency fixing period which is longer than the frame time. Hereinafter, the operation of the measuring section 156 will be described in detail.

The measuring section 156 identifies the distance between the biological condition measurement apparatus 1 and the measurement subject U on the basis of the propagation time from the transmission of the transmission signal by the transmitting section 11 until the reception of the reflection signal by the receiving section 12. The measuring section 156 identifies the direction of the measurement subject U on the basis of a combination of a transmitting section 11 that has transmitted a transmission signal and a receiving section 12 that has received a reflection signal. The measuring section 156 identifies the cycle of motions of body parts of the measurement subject U on the basis of the cycle of the change in the propagation time.

The measuring section 156 identifies the movement speed of the measurement subject U on the basis of the amount of change in the propagation time and the amount of change in the direction of the measurement subject U. The measuring section 156 can also identify the movement speed of the measurement subject U by calculating angular frequencies on the basis of the magnitudes of changes of the phases of the reflection signals based on chirp signals transmitted at a certain time intervals.

Although any method can be used by the measuring section 156 to identify propagation times on the basis of reflection signals, the measuring section 156 identifies a frequency component selected by the frequency selection section 155 from among a plurality of frequency components included in a chirp signal included in the reflection signal in the frequency domain, and identifies, as a propagation time, a difference between a time at which a transmitting section 11 transmitted the frequency component and a time at which a receiving section 12 received the frequency component included in the reflection signal. In order to improve the measurement precision, the measuring section 156 may measure a propagation time by identifying propagation times of a plurality of frequency components corresponding to the plurality of frequency components that are selected by the frequency selection section 155, and calculating a statistic (e.g. the average or the median) of a plurality of the identified propagation times.

FIG. 7 illustrates an operation of the measuring section 156. White oblongs in FIG. 7 represent transmission signals transmitted by the transmitting sections 11, and black oblongs in FIG. 7 represent reflection signals received by the receiving sections 12. In the example depicted in FIG. 7, a transmitting section 11A, a transmitting section 11B and a transmitting section 11C sequentially transmit transmission signals at times T1, T2 and T3, and subsequently the transmitting section 11A, the transmitting section 11B and the transmitting section 11C sequentially transmit transmission signals at times T4, T5 and T6.

As depicted in FIG. 7, cycles in which the transmitting sections 11 transmit transmission signals are set to have a length of time that is longer than ((time length of transmission signals) + (length of time during which reflection signals may be received)) × (number of transmitting sections 11). By determining the cycles in which transmission signals are transmitted in this manner, it is possible to prevent simultaneous arrivals of reflection signals of transmission signals transmitted by the plurality of transmitting sections 11 at the biological condition measurement apparatus 1.

The measuring section 156 measures a length of time that elapses from the transmission of a transmission signal by each of the transmitting section 11A, the transmitting section 11B and the transmitting section 11C until reception of a reflection signal. In the case of the example depicted in FIG. 7, the measuring section 156 measures reception of a reflection signal by the receiving section 12A after a lapse of a length of time D1 after the transmission of the transmission signal by the transmitting section 11A at the time T1. The reflection signal is a reflection signal generated by reflection of the transmission signal transmitted by the transmitting section 11A on the measurement subject U1 in the example depicted in FIG. 5.

Also, the measuring section 156 measures reception of a reflection signal by the receiving section 12A also after a lapse of a length of time D2 after the transmission of the transmission signal by the transmitting section 11A. The reflection signal is a reflection signal generated by reflection of the transmission signal transmitted by the transmitting section 11A on a measurement subject U2, who is a measurement subject U different from the measurement subject U1 in the example depicted in FIG. 5. Furthermore, the measuring section 156 measures reception of a reflection signal by the receiving section 12C also after a lapse of a length of time D3 after the transmission of the transmission signal by the transmitting section 11A. The reflection signal is a reflection signal generated by reflection of the transmission signal transmitted by the transmitting section 11A on a measurement subject U3 in the example depicted in FIG. 5.

Similarly, the measuring section 156 measures reception of a reflection signal by the receiving section 12A after a lapse of a length of time D4 after the transmission of the transmission signal by the transmitting section 11B. The reflection signal is a reflection signal generated by reflection of the transmission signal transmitted by the transmitting section 11B on a measurement subject U4 in the example depicted in FIG. 5.

The measuring section 156 repeats similar processes also after the time T4. As depicted in FIG. 7, the propagation times D1 to D4 from the transmission of the transmission signals by the transmitting sections 11 until the reception of the reflection signals by the receiving sections 12 changed to different D1' to D4'. This is caused by changes of the distances between parts that reflected the transmission signals on the measurement subject U1 to the measurement subject U4 and the biological condition measurement apparatus 1 due to motions of said parts.

The measuring section 156 may further measure the magnitude of intensities of reflection signals received by receiving sections 12, and store, in the storage section 14, the measured magnitude of intensities of the reflection signals together with the propagation times. Since attenuation amounts of the signals vary depending on the distances between the biological condition measurement apparatus 1 and measurement subjects U, as depicted in FIG. 7, it is considered that the intensities of reflection signals decrease as the propagation times increase. In a case where the intensity of a reflection signal with a long propagation time is higher than the intensity of a reflection signal with a short propagation time, the measuring section 156 may determine that the reflection signals may include noise, and not store the propagation times of the reflection signals in the storage section 14.

The measuring section 156 identifies biological conditions of each of a plurality of measurement subjects U on the basis of propagation times measured by the measuring section 156. In a case where the biological condition measurement apparatus 1 has a plurality of transmitting sections 11, the measuring section 156 identifies biological conditions of a plurality of measurement subjects U in areas toward which the plurality of transmitting sections 11 transmit transmission signals. In a case where the biological condition measurement apparatus 1 has a plurality of receiving sections 12, the measuring section 156 identifies biological conditions of a plurality of measurement subjects in areas from which the plurality of receiving sections 12 receive reflection signals.

The measuring section 156 identifies the biological conditions of the measurement subject U on the basis of changes in the phase of the frequency component corresponding to the frequency stored in the storage section 14. The measuring section 156 generates a phase signal by arranging a plurality of pieces of phase data indicating the phases of the frequency component corresponding to the frequency selected by the frequency selection section 155 in time series over the frequency fixing period. Next, the measuring section 156 identifies the biological conditions on the basis of a frequency component included in a cycle range or frequency range corresponding to the biological conditions in the generated phase signal.

The measuring section 156 identifies the respiratory rate and heart rate by identifying a time interval between peaks in the phase signal, for example. When the time interval between the peaks falls within the range assumed as a cycle of biological conditions to be identified, the measuring section 156 identifies the identified time interval as the cycle of the biological conditions. The measuring section 156 identifies the respiration rate per unit time on the basis of the time interval between peaks within the range assumed to be the respiratory cycle. Similarly, the measuring section 156 identifies the heart rate per unit time on the basis of the time interval between the peaks within the range assumed to be the heart rate cycle. The range assumed to be the respiratory cycle is, for example, 3 seconds or more and 6 seconds or less, and the range assumed to be the heart rate cycle is, for example, 0.5 seconds or more and 1 second or less.

The measuring section 156 may convert a signal generated by arranging the phases of the frequency component corresponding to the frequency selected by the frequency selection section 155 in time series into a frequency-domain signal via an FFT process, and separate the signal into a frequency component included in a respiratory frequency band and a frequency component included in a heartbeat frequency band by a band-pass filter. The respiration frequency band corresponds to a range assumed to be the frequency of respiration (that is, the reciprocal of cycle), and is 0.167 Hz or more and 0.333 Hz or less (corresponding to the respiratory rate between 10 and 20 breaths per minute), for example. The heartbeat frequency band corresponds to a range assumed to be the frequency of the heart rate (that is, the reciprocal of cycle), and is 1 Hz or more and 2 Hz or less (the heart beats per minute is between 60 to 120 times), for example. These values are stored in the storage section 14 after the operation accepting section 151 accepts the setting, for example.

The measuring section 156 identifies, as the respiratory frequency, a frequency of a frequency component with an intensity equal to or higher than a first intensity (for example, a frequency component with the highest intensity) among a plurality of frequency components included in the respiratory frequency band. Similarly, the measuring section 156 identifies, as the heartbeat frequency, a frequency of a frequency component with an intensity equal to or higher than a second intensity (for example, a frequency component with the highest intensity) among a plurality of frequency components included in the heartbeat frequency band. By having the measuring section 156 operate in this manner, it is possible to identify the frequencies of respiration and heart rate with high accuracy.

In a case where the frequency selected by the frequency selection section 155 is switched at each frequency fixing period, the time-series data may change discontinuously before and after the switching of the frequency. In such a case, the measuring section 156 may correct the phase signal so as to reduce the discontinuity of the phase signal based on the time-series phase data at the timing of the frequency switching. The measuring section 156 corrects, for example, a value of phase data within a predetermined range from the end of the time-series phase data before the frequency switching and a value of phase data within a predetermined range from the head of the time-series phase data after the frequency switching so that the phase signal continuously changes.

Due to the low intensity of the signal reflected on the heart, the measuring section 156 may amplify the frequency component included in the heartbeat frequency band. The measuring section 156 may amplify the reflection signal using different amplification factors for the respiratory frequency band and the heartbeat frequency band.

The measuring section 156 may identify the biological conditions by extracting a plurality of samples (M samples) for a fixed period from the signal generated by arranging the phases of the frequency component corresponding to the frequency selected by the frequency selection section 155 in time series, and performing a matching process on the plurality of samples to identify the cycle of said signal. In the matching process, the measuring section 156 sets the sum of squared differences of the plurality of samples shifted by a predetermined sampling time as an evaluation value, and identifies a shift amount for which the evaluation value becomes minimum as the cycle of said signal, for example.

The measuring section 156 identifies the heart rate on the basis of the phase signal over the frequency fixing period, and identifies the respiratory rate on the basis of the phase signal that spans 1.5 times or more and 2.5 times or less the frequency fixing period. When the frequency fixing period is set to, for example, 5 seconds or more and 10 seconds or less, by having the measuring section 156 operate in this manner, the heart rate and respiratory rate can be measured on the basis of the phase signals over a plurality of cycles of the heart rates and respiration, thereby improving the measurement precision.

In a case where the biological condition measurement apparatus 1 has a plurality of transmitting sections 11 and a plurality of receiving sections 12, the measuring section 156 identifies biological conditions of a plurality of measurement subjects U in each of a plurality of areas that are determined from combinations of areas toward which the plurality of transmitting sections 11 transmit transmission signals and areas from which the plurality of receiving sections 12 receive reflection signals, on the basis of measured propagation times for each combination of a transmitting section 11 and a receiving section 12. By being configured in this manner, the biological condition measurement apparatus 1 can identify biological conditions of each measurement subject U even in a case where there are a plurality of measurement subjects U at the same distance from the biological condition measurement apparatus 1.

In order to identify biological conditions of a plurality of measurement subjects U, the measuring section 156 creates waveforms representing cardiac motions, waveforms representing pleural motions or the like first by identifying changes of the distance between the biological condition measurement apparatus 1 and a part that reflected transmission signals on each of the plurality of measurement subjects U, on the basis of changes of measured propagation times. By identifying the cycles of the waveforms, the measuring section 156 identifies biological conditions such as a heart rate or a respiratory rate per unit time.

As depicted in FIG. 7, in a case where transmission signals reflected on a plurality of measurement subjects U, a receiving section 12 receives a plurality of reflection signals based on one transmission signal. The measuring section 156 performs a process in the following manner in order to identify a propagation time corresponding to each of a plurality of measurement subjects U.

First, the measuring section 156 classifies propagation time data representing a plurality of measured propagation times into a plurality of pieces of time series data including a plurality of pieces of propagation time data representing propagation times that fluctuate within a time range equal to or lower than a first threshold, and identifies biological conditions of each of the plurality of measurement subjects U on the basis of changes in each time interval of propagation times represented by each of the plurality of pieces of time series data. The first threshold is a value that is higher than the maximum value of change amounts of propagation times caused by motions of an organ of one measurement subject U, and additionally is smaller than an expected minimum value of differences from propagation times of reflection signals from other measurement subjects U. The measuring section 156 stores, in the storage section 14, the identified biological conditions in association with the measurement subjects U.

The maximum value of change amounts of propagation times caused by motions of an organ of a measurement subject U corresponds to an expected greatest amount (e.g. 5 cm) of amounts of displacement of the position of the organ in a stationary state of the measurement subject U, and the differences from the propagation times of the reflection signals from the other measurement subjects U correspond to a distance difference of 50 cm, for example. That is, the first threshold is a length of time required for radio waves to be propagated over any distance that is equal to or longer than 5 cm and is shorter than 50 cm.

FIG. 7 depicts a case where the difference between the propagation times of the reflection signal whose delay from the time T1 is D1 and the reflection signal whose delay from the time T4 which is a predetermined time interval after the time T1 is D1' is shorter than the first threshold. The measuring section 156 determines that these reflection signals are reflection signals corresponding to the same measurement subject U. In addition, FIG. 7 depicts a case where the difference between the propagation times of the reflection signal whose delay from the time T1 is D1 and the reflection signal whose delay from the time T4 is D2' is equal to or higher than the first threshold. The measuring section 156 determines that these reflection signals are reflection signals corresponding to mutually different measurement subjects U.

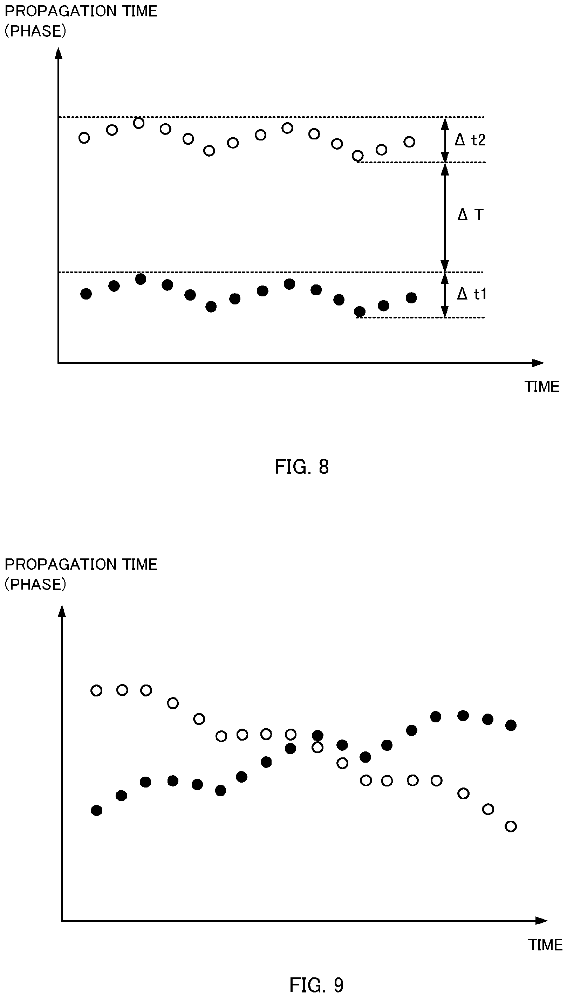

FIG. 8 illustrates the operation of the measuring section 156. The horizontal axis in FIG. 8 represents times, and the vertical axis in FIG. 8 represents propagation times of the reflection signals measured by the measuring section 156 at each time. The fluctuation of the propagation times is equivalent to the fluctuation of the phase of the reflection signal. Black circles in FIG. 8 represent the propagation times of reflection signals corresponding to the measurement subject U1, and white circles in FIG. 8 represent the propagation times of reflection signals corresponding to the measurement subject U2. The extent of fluctuation of a plurality of the propagation times represented by the black circles in one cycle is Δt1, the extent of fluctuation of a plurality of the propagation times represented by the white circles in one cycle is Δt2, and these are shorter than the first threshold. On the other hand, the minimum value ΔT of differences between the propagation times represented by the black circles and the propagation times represented by the white circles is equal to or higher than the first threshold.

The propagation time of the reflection signal corresponding to the measurement subject U1 is a length of time that elapses from transmission of a first frequency component included in the transmission signal by the transmitting section 11 until reception of a first frequency component included in the reflection signal by the receiving section 12. The propagation time of the reflection signal corresponding to the measurement subject U2 is a length of time that elapses from transmission of a second frequency component included in the transmission signal by the transmitting section 11 until reception of a second frequency component included in the reflection signal by the receiving section 12.

The measuring section 156 classifies the plurality of pieces of propagation time data into first time series data corresponding to the plurality of propagation times represented by the black circles and second time series data corresponding to the plurality of propagation times represented by the white circles. Then, the measuring section 156 identifies biological conditions of the measurement subject U1 on the basis of the first time series data, and identifies biological conditions of the measurement subject U2 on the basis of the second time series data. By operating in this manner, the measuring section 156 can identify biological conditions of each of a plurality of measurement subjects U even in a case where receiving sections 12 received a plurality of reflection signals based on a transmission signal transmitted by one transmitting section 11.

After identifying the biological conditions of the plurality of measurement subjects U, the measuring section 156 may track the biological conditions of the measurement subjects U who are moving, on the basis of propagation times. The measuring section 156 identifies, as biological conditions of one measurement subject U, time series data including a plurality of pieces of propagation time data representing propagation times that fluctuate in a time range equal to or lower than a second threshold higher than the first threshold in cycles during which the propagation times fluctuate, and stores, in the storage section 14, the identified biological conditions in association with the measurement subject U. The second threshold is a value that is higher than the maximum value of change amounts of propagation times in a predetermined length of time (e.g. in one cycle during which propagation times fluctuate) caused by a movement of one measurement subject U, and additionally is smaller than the differences from propagation times of reflection signals from other measurement subjects U.

FIG. 9 illustrates a process in which the measuring section 156 tracks biological conditions of the measurement subject U. Similarly to FIG. 8, black circles in FIG. 9 represent the propagation times of reflection signals corresponding to the measurement subject U1, and white circles in FIG. 9 represent the propagation times of reflection signals corresponding to the measurement subject U2.

FIG. 9 depicts a case where the measurement subject U1 and the measurement subject U2 are moving, and the propagation times change over time. Specifically, the measurement subject U1 is moving away from the biological condition measurement apparatus 1 with time, and the measurement subject U2 is moving toward the biological condition measurement apparatus 1 with time. The measuring section 156 identifies first time series data corresponding to the measurement subject U1 represented by black, and second time series data corresponding to the measurement subject U2 represented by white by selecting a plurality of pieces of propagation time data representing propagation times whose fluctuation ranges are equal to or shorter than the second threshold for each fluctuation cycle of propagation times in a plurality of pieces of propagation time data.

Since the measuring section 156 generates the first time series data on the basis of the first frequency component and generates the second time series data on the basis of the component of the second frequency, the first time series data and the second time series data can be separated even at a point in time when the positions of the measurement subject U1 and the measurement subject U2 become almost the same.

However, as a result of the frequency selection section 155 reselecting the frequency, the frequency corresponding to the measurement subject U1 may be changed from the first frequency to the second frequency, and the frequency corresponding to the measurement subject U2 may be changed from the second frequency to the first frequency. In such a case, at a point in time when the positions of the measurement subject U1 and the measurement subject U2 become almost the same, the measuring section 156 may identify propagation time data corresponding to each of the measurement subjects U on the basis of features of waveforms (e.g. intensities or cycles) formed by linking the preceding and following pieces of propagation time data. Specifically, in case where it is unknown to which measurement subject U propagation time data corresponds, the measuring section 156 assumes that the propagation time data is propagation time data of either measurement subject U, and identifies features of a waveform of a cycle including the propagation time data. In a case where the identified features match features of the past cycles, the measuring section 156 determines that the assumption is correct, and in a case where the identified features do not match features of the past cycles, the measuring section 156 determines that the assumption is wrong, and identifies that the propagation time data corresponds to another measurement subject U.

The measuring section 156 may identify biological conditions corresponding to each of a plurality of parts of the measurement subject U. For example, the measuring section 156 identifies the parts on the basis of the propagation times each having different frequency components included in a plurality of reflection signals generated by reflection of transmission signals on a plurality of parts like cardiac motions, carotid artery motions, pleural motions, head motions or the like of the measurement subject U. By classifying a plurality of the reflection signals generated by the reflection of one transmission signal transmitted by a transmitting section 11 on the plurality of parts of the measurement subject U on the basis of a combination of a range in which propagation times measured by the measuring section 156 are included and the frequency selected by the frequency selection section 155, the measuring section 156 identifies a plurality of biological conditions corresponding to the plurality of parts. By the measuring section 156 operating in this manner, a plurality of biological conditions of the measurement subject U can be identified even when a difference among each of propagation times corresponding to the plurality of parts is small.

The measuring section 156 may identify the positions of a plurality of measurement subjects U. Specifically, the measuring section 156 identifies the positions of measurement subjects U that generated reflection signals on the basis of: areas from which receiving sections 12 that received the reflection signals receive the reflection signals; areas toward which transmitting sections 11 that transmitted transmission signals to generate the reflection signals transmit the transmission signals; and the distances to the measurement subjects U corresponding to propagation times.

In the example depicted in (a) in FIG. 5, a transmission signal transmitted by the transmitting section 11A is reflected on the measurement subject U1, the measurement subject U2 and the measurement subject U3, and a transmission signal transmitted by the transmitting section 11B is reflected on the measurement subject U4. Then, reflection signals generated by the reflection on the measurement subject U1, the measurement subject U2 and the measurement subject U4 are received by the receiving section 12A, and a reflection signal generated by the reflection on the measurement subject U3 is received by the receiving section 12C.

In a case where reflection signals based on the transmission signal transmitted by the transmitting section 11A are received by the receiving section 12A, the measuring section 156 identifies that the measurement subject U1 and the measurement subject U2 that reflected the transmission signal are in a region where the transmission area of the transmitting section 11A and the reception area of the receiving section 12A overlap. In addition, in a case where a reflection signal based on the transmission signal transmitted by the transmitting section 11A is received by the receiving section 12C, the measuring section 156 identifies that the measurement subject U3 that reflected the transmission signal is in a region where the transmission area of the transmitting section 11A and the reception area of the receiving section 12C overlap.

The measuring section 156 further identifies the distances between the biological condition measurement apparatus 1 and measurement subjects U on the basis of propagation times. The measuring section 156 can identify the positions of a plurality of measurement subjects U relative to the biological condition measurement apparatus 1 on the basis of regions where the measurement subjects U are and the distances from the biological condition measurement apparatus 1 to the measurement subjects U.

The output section 155 outputs, in association with each other, biological conditions and information representing the positions of the measurement subjects U identified by the measuring section 156. Specifically, the output section 155 causes the information terminal 2 to display the screen representing the biological conditions depicted in FIG. 2 and the screen representing the positions of the measurement subjects U depicted in FIG. 3, outputs these pieces of data to a printer, transmits these pieces of data to an external apparatus via a communication line, and so on.

As an example, as depicted in FIG. 2, the output section 155 prioritizes output of biological conditions corresponding to a measurement subject U identified as being the closest to the biological condition measurement apparatus 1 on the basis of propagation times in a plurality of biological conditions corresponding to a plurality of measurement subjects U identified by the measuring section 156, over output of biological conditions corresponding to other measurement subjects U. For example, the output section 155 causes the information terminal 2 to display biological conditions sequentially starting from a measurement subject U closer to the biological condition measurement apparatus 1, causes the information terminal 2 to display only biological conditions of a measurement subject U closest to the biological condition measurement apparatus 1, and so on. By the output section 155 operating in this manner, in a case where there are many humans in a room where the biological condition measurement apparatus 1 is used, it becomes easier for a user to grasp biological conditions of measurement subjects U whose biological conditions are desired to be measured.

### [Processing procedure in the biological condition measurement apparatus 1]

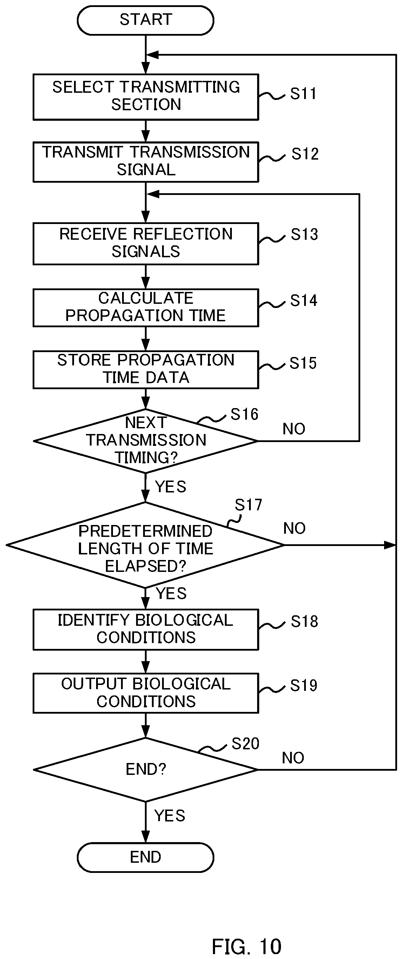

FIG. 10 is a flowchart showing an outline of a processing procedure in the biological condition measurement apparatus 1. The flowchart depicted in FIG. 10 starts at a time point when a user of the biological condition measurement apparatus 1 performed operation to start measurement.

The radio-wave control section 152 selects a transmitting section 11 to be caused to transmit a transmission signal from a plurality of transmitting sections 11 on the basis of a time (S11). In the case of the example depicted in FIG. 7, the radio-wave control section 152 selects the transmitting section 11A at the time T1. The radio-wave control section 152 causes the selected transmitting section 11 to transmit a transmission signal (S12).

Next, a receiving section 12 receives reflection signals based on the transmission signal (S13). The measuring section 156 calculates a propagation time required for the reflection signals to be received by the receiving section 12 after the transmission of the transmission signal (S14). The measuring section 156 stores, in the storage section 14, propagation time data in association with the combination of the transmitting section 11 that transmitted the transmission signal and the receiving section 12 that received the reflection signals (S15).

The radio-wave control section 152 determines whether or not the next transmission timing (e.g. the time T2) has come (S16). In a case where the radio-wave control section 152 determined that the next transmission timing has not come (NO at S16), the radio-wave control section 152 does not cause any transmitting section 11 to transmit a transmission signal, and the processes from S13 to S16 are repeated.

In a case where the radio-wave control section 152 determined that the next transmission timing has come (YES at S16), the measuring section 156 determines whether or not a predetermined length of time has elapsed after the start of transmission of transmission signals by transmitting sections 11, and a timing to identify biological conditions has come (S17). The predetermined length of time is a length of time required for storing a number of pieces of propagation time data necessary for the measuring section 156 to identify biological conditions. The predetermined length of time is a frequency fixing period longer than one cycle of biological conditions, and is equal to or longer than 5 seconds, for example.

In a case where the measuring section 156 determined that the predetermined length of time has not elapsed (NO at S17), the radio-wave control section 152 selects the next transmitting section 11 (S11), and the processes from S11 to S17 are repeated. In a case where the measuring section 156 determined that the predetermined length of time has elapsed (YES at S17), the measuring section 156 identifies biological conditions on the basis of the propagation time data stored in the storage section 14 (S18). The output section 155 outputs the biological conditions identified by the measuring section 156 (S19). The biological condition measurement apparatus 1 repeats the processes from S11 to S20 until the operation accepting section 151 accepts operation to end the processes (NO at S20).

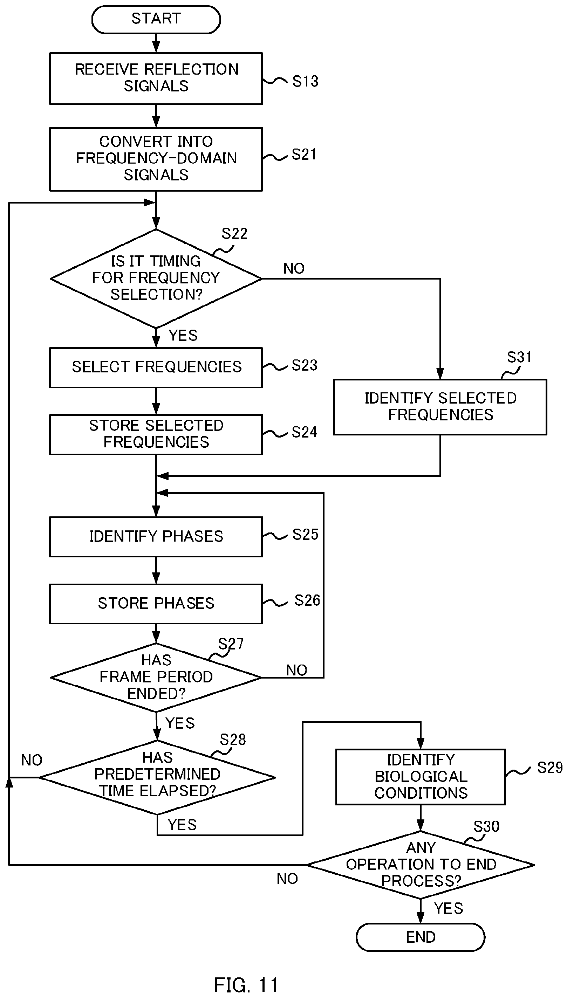

FIG. 11 is a flowchart showing details of a process of analyzing the reflection signals in the biological condition measurement apparatus 1. The flowchart illustrated in FIG. 11 starts at a time point when the receiving section 12 receives reflection signals (S13 in FIG. 10).

When the receiving section 12 receives the reflection signals, the signal converting section 154 converts the reflection signals into frequency-domain signals using the FFT (S21). Next, the frequency selection section 155 determines whether it is the timing to select frequencies of the frequency components to be analyzed (S22). That is, the frequency selection section 155 determines whether the frequency fixing period has elapsed since the previous frequency was reselected.

If it is determined that it is the timing for frequency selection (YES in S22), the frequency selection section 155 selects a predetermined number of frequencies on the basis of the magnitudes of intensities (that is, amplitude or power) of the plurality of frequency components (S23). The frequency selection section 155 stores data indicating the selected frequencies in the storage section 14 (S24).

Next, the measuring section 156 identifies the phases of the frequency components of the selected frequencies (S25). The measuring section 156 stores the identified phases in the storage section 14 in association with the timing (S26). The measuring section 156 repeats the processes of S25 and S26 until one frame period of the FFT ends (during NO in S27).

When the frame period ends (YES in S27), the measuring section 156 determines whether a predetermined time required for identifying the biological conditions has elapsed (S28). If the predetermined time has elapsed (YES in S28), the measuring section 156 identifies the biological conditions (S29). If the predetermined time has not elapsed (NO in S28), the measuring section 156 returns the process to S22. The predetermined time is at least twice the maximum value of a value assumed as the cycle of the biological condition. If the biological condition is respiratory rate, the predetermined time is, for example, 12 seconds or more (preferably 60 seconds), and if the biological condition is heart rate, the predetermined time is, for example, 2 seconds or more (preferably 10 seconds).

The measuring section 156 determines whether the operation accepting section 151 has received an operation to end the process (S30). If the end operation has not been performed (NO in S30), the measuring section 156 returns the process to S22.

If it is determined in S22 that it is not the timing to select the frequencies (NO in S22), the frequency selection section 155 identifies the selected frequencies stored in the storage section 14 in S24 (S31). The measuring section 156 analyzes the frequency components corresponding to the frequencies included in the reflection signals to execute the processes from S25 to S29.

### [Modification Example]

Whereas the biological condition measurement apparatus 1 identifies biological conditions of a plurality of measurement subjects U in the case illustrated in the explanation above, some of the functions of the control section 15 of the biological condition measurement apparatus 1 in the explanation described above may be realized by a second information processing apparatus (e.g. a computer in a cloud). That is, the biological condition measurement apparatus 1 and the second information processing apparatus may operate in a coordinated manner to thereby function as a biological condition measurement system.

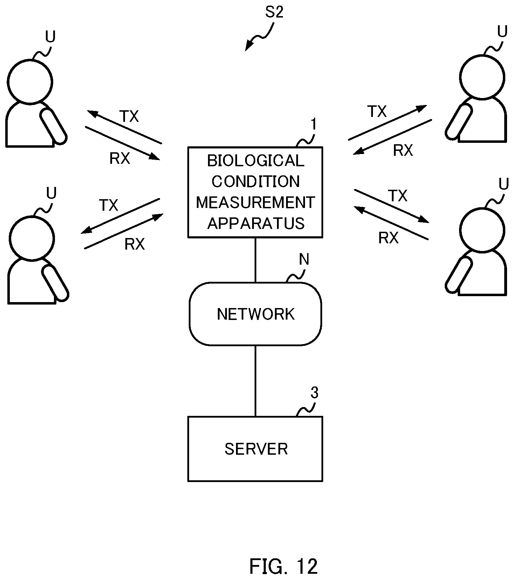

FIG. 12 shows a configuration of a biological condition measurement system S2 according to a modification example. For example, the biological condition measurement apparatus 1 of the biological condition measurement system S sequentially transmits received reflection signals to an external server 3. In this case, the external server 3 executes at least some of the functions of the signal acquiring section 153, the signal converting section 154, the frequency selection section 155, and the measuring section 156. Specifically, the external server 3 classifies reflection signals of each of a plurality of measurement subjects U, identifies biological conditions on the basis of propagation times, and transmits results of the identification to any terminal.

The biological condition measurement apparatus 1 may transmit data of the frequency components corresponding to the frequencies selected by the frequency selection section 155 to the external server 3, and the server 3 may execute the process of the measuring section 156. In addition, the biological condition measurement apparatus 1 may transmit, to the server 3, only data of the frequency components included in the reflection signals received by the receiving section 12 that corresponds to the position where the presence of the measurement subject U is detected. With such configurations, the amount of data to be transmitted to the server 3 as well as the computational load on the server 3 can be reduced.

The external server 3 may receive reflection signals from a plurality of the biological condition measurement apparatuses 1, and identify biological conditions of a plurality of measurement subjects U corresponding to the reflection signals received by the plurality of biological condition measurement apparatuses 1. Such configuration is suitable for a case where there are many measurement subjects U in a plurality of rooms.

### [Effects attained with the biological condition measurement apparatus 1]

As explained above, the frequency selection section 155 selects frequencies with relatively high intensities of the plurality of frequency components corresponding to each of the plurality of frequencies included in the reflection signal each time the frequency fixing period elapses. The measuring section 156 can measure the biological conditions such as the heart rate or respiratory rate of the measurement subject U by identifying the fluctuation cycle of the phase of the frequency components of the selected frequency in the reflection signal while suppressing the computational load.

The present invention is explained based on the exemplary embodiments. The technical scope of the present invention is not limited to the scope explained in the above embodiments and it is possible to make various changes and modifications within the scope of the disclosure. For example, all or part of the apparatus can be configured with any unit which is functionally or physically dispersed or integrated. Further, new exemplary embodiments generated by arbitrary combinations of them are included in the exemplary embodiments. Further, effects of the new exemplary embodiments brought by the combinations also have the effects of the original exemplary embodiments.

### [Description of symbols]

1 Biological condition measurement apparatus
2 Information terminal
3 Server
11 Transmitting section
12 Receiving section
13 External connection section
14 Storage section
15 Control section
151 Operation accepting section
152 Radio-wave control section
153 Signal acquiring section
154 Signal converting section
155 Frequency selection section
156 Measuring section

## Claims

1. A biological condition measurement apparatus comprising:
a signal acquiring section that acquires a reflection signal generated by reflection, on a measurement subject, of a chirp signal in a frequency band equal to or higher than a millimeter wave band, transmitted at each predetermined frame time;
a signal converting section that converts the reflection signal into a complex signal in a frequency domain for each frame time;
a frequency selection section that selects a frequency corresponding to a frequency component with relatively high intensity from among a plurality of frequency components included in the complex signal; and
a measuring section that identifies biological conditions of the measurement subject on the basis of changes in a phase of the frequency component corresponding to the frequency selected by the frequency selection section over a frequency fixing period which is longer than the frame time.

2. The biological condition measurement apparatus according to claim 1, wherein
the measuring section generates a phase signal by arranging phases of the frequency component in time series over the frequency fixing period, and identifies the biological conditions on the basis of a frequency component included in a cycle range or a frequency range corresponding to the biological conditions in the phase signal.

3. The biological condition measurement apparatus according to claim 2, wherein
the measuring section identifies a time interval between peaks in the phase signal as a cycle of the biological conditions when the time interval falls within a range assumed as the cycle of the biological condition to be identified.

4. The biological condition measurement apparatus according to claim 2, wherein
the measuring section converts the phase signal into a frequency-domain signal, identifies, as respiratory frequency, a frequency of a frequency component with a first intensity or higher among a plurality of frequency components included in a respiratory frequency band, and identifies, as a heartbeat frequency, a frequency of a frequency component with a second intensity or higher among a plurality of frequency components included in a heartbeat frequency band.

5. The biological condition measurement apparatus according to claim 2, wherein
the measuring section identifies a heart rate on the basis of the phase signal over the frequency fixing period, and identifies a respiratory rate on the basis of the phase signal that spans 1.5 times or more and 2.5 times or less the frequency fixing period.

6. The biological condition measurement apparatus according to claim 5, wherein
the frequency fixing period is 5 seconds or more and less than 10 seconds.

7. The biological condition measurement apparatus according to claim 2, wherein
the measuring section corrects the phase signal so as to reduce discontinuity of the phase signal at a timing at which a frequency selected by the frequency selection section is switched.

8. The biological condition measurement apparatus according to claim 1, wherein
the frequency selection section selects a frequency corresponding to a frequency component with relatively high intensity at each frequency fixing period.

9. The biological condition measurement apparatus according to claim 1, wherein
the frequency selection section selects a frequency corresponding to a frequency component with the highest intensity from among the plurality of frequency components.

10. The biological condition measurement apparatus according to claim 1, wherein
the signal acquiring section acquires the reflection signal generated by reflection on a plurality of the subjects; and
the frequency selection section selects a frequency corresponding to the number of frequency components corresponding to the number of measurement subjects from among the plurality of frequency components.

11. The biological condition measurement apparatus according to claim 1, wherein
the biological condition measurement apparatus comprises a storage section that stores a frequency corresponding to the frequency component selected by the frequency selection section, and
the measuring section identifies the biological conditions of the measurement subject on the basis of changes in the phase of the frequency component corresponding to the frequency stored in the storage section.

12. A biological condition measurement method executed by a computer and comprising the steps of:
acquiring a reflection signal generated by reflection, on a measurement subject, of a chirp signal in a frequency band equal to or higher than a millimeter wave band, transmitted at each predetermined frame time;
a signal converting section that converts the reflection signal into a complex signal in a frequency domain for each frame time;
selecting a frequency corresponding to a frequency component with a relatively high intensity from among a plurality of frequency components included in the complex signal; and
identifying biological conditions of the measurement subject on the basis of changes in a phase of the frequency component corresponding the selected frequency over a frequency fixing period which is longer than the frame time.

13. A program for causing a computer to execute the steps of:
acquiring a reflection signal generated by reflection, on a measurement subject, of a chirp signal in a frequency band equal to or higher than a millimeter wave band, transmitted at each predetermined frame time;
a signal converting section that converts the reflection signal into a complex signal in a frequency domain for each frame time;
selecting a frequency corresponding to a frequency component with a relatively high intensity from among a plurality of frequency components included in the complex signal; and
identifying biological conditions of the measurement subject on the basis of changes in a phase of the frequency component corresponding to the selected frequency over a frequency fixing period which is longer than the frame time.

14. A biological condition measurement system comprising:
a biological condition measurement apparatus; and
an external apparatus that can communicate with the biological condition measurement apparatus, wherein
the biological condition measurement apparatus comprises: a signal acquiring section that acquires a reflection signal generated by reflection, on a measurement subject, of a chirp signal in a frequency band equal to or higher than a millimeter wave band, transmitted at each predetermined frame time;
the biological condition measurement apparatus or the external apparatus has:
a signal converting section that converts the reflection signal into a complex signal in a frequency domain for each frame time;
a frequency selection section that selects a frequency corresponding to a frequency component with relatively high intensity from among a plurality of frequency components included in the complex signal; and
a measuring section that identifies biological conditions of the measurement subject on the basis of changes in a phase of the frequency component corresponding to the frequency selected by the frequency selection section over a frequency fixing period which is longer than the frame time.
